(19) **Europäisches Patentamt / European Patent Office / Office européen des brevets**

(11) **EP 1 750 143 A2**

(12) **EUROPEAN PATENT APPLICATION**

(43) Date of publication:
**07.02.2007 Bulletin 2007/06**

(51) Int Cl.:
***G01S 15/89*** *(2006.01)*

(21) Application number: **06015539.7**

(22) Date of filing: **26.07.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **27.07.2005 KR 20050068260**

(71) Applicant: **MEDISON CO., LTD.**
**Kangwon-do 250-870 (KR)**

(72) Inventors:
• **KIM, Jae Gyoung,**
**Discusser & Medison Building,**
**Seoul 135-280 (KR)**

• **SONG, Young Seuk,**
**Discusser & Medison Building,**
**Seoul 135-280 (KR)**
• **CHOI, Do Young,**
**Discusser & Medison Building,**
**Seoul 135-280 (KR)**

(74) Representative: **Lorenz, Markus**
**Lorenz & Kollegen**
**Alte Ulmer Strasse 2**
**89522 Heidenheim (DE)**

(54) **Ultrasound diagnostic system and method of automatically controlling brightness and contrast of a three-dimensional ultrasound image**

(57) The present invention relates to an ultrasound diagnostic system and method for automatically controlling the brightness and contrast of a three-dimensional (3D) ultrasound image. The method for automatically controlling the brightness and contrast of a 3D ultrasound image includes the steps of: creating 3D ultrasound image data based on ultrasound echo signals; setting a critical value for rendering the 3D ultrasound image data; rendering the 3D ultrasound image data by using the critical value to form a 3D ultrasound image; analyzing a histogram of the 3D ultrasound image to set image parameters for the 3D ultrasound image; and adjusting the brightness and contrast of the 3D ultrasound image based on the image parameters.

FIG. 1

100

EP 1 750 143 A2

**Description**

The present invention generally relates to an ultrasound imaging system, and more particularly to an ultrasound diagnostic system and method for automatically controlling the brightness and contrast of a three-dimensional ultrasound image.

An ultrasound diagnostic system has become an important and popular diagnostic tool due to its wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., organs of a human patient). The ultrasound diagnostic system generally uses a wide bandwidth transducer to transmit and receive ultrasound signals. The ultrasound diagnostic system forms images of the internal tissues of a human body by electrically exciting an acoustic transducer element or an array of acoustic transducer elements to generate ultrasound pulses that travel into the body. The ultrasound pulses produce ultrasound echoes since they are reflected from body tissues, which appear as discontinuities to the propagating ultrasound pulses. The various ultrasound echoes return to the transducer and are converted into electrical signals, which are amplified and processed to produce ultrasound data for an image of the tissues. The ultrasound diagnostic system is of significant importance to the medical field since it provides physicians with real-time high-resolution images of internal features of a human anatomy without the need for invasive observation techniques such as surgery.

Medical ultrasound images have traditionally been presented as two-dimensional (2D) images of essentially raw ultrasound data. Recently, three-dimensional (3D) ultrasound imaging technologies have been developed to overcome the limitations of 2D ultrasound images and to increase the ultrasound's overall clinical efficacy. To readily distinguish a target object from other objects (e.g., background objects), the user of the ultrasound diagnostic system has to finely adjust image parameters such as the brightness and contrast of the 3D ultrasound image displayed on a screen. However, in the conventional ultrasound diagnostic system, the image parameters are adjusted manually (not automatically). That is, a complicated manual adjustment is required to optimize the 3D ultrasound image. Thus, the diagnostic time becomes longer.

The present provides an ultrasound diagnostic system and method for automatically controlling the brightness and contrast of a three-dimensional (3D) ultrasound image to optimize the 3D ultrasound image and to reduce diagnostic time by minimizing the operations of a system user.

According to one aspect of the present invention, there is provided a method of automatically controlling the brightness and contrast of a three-dimensional (3D) ultrasound image, which comprises the following steps: a) creating 3D ultrasound image data based on ultrasound echo signals; b) setting a critical value for rendering the 3D ultrasound image data; c) rendering the 3D ultrasound image data by using the critical value to form a 3D ultrasound image; d) analyzing a histogram of the 3D ultrasound image to set image parameters for the 3D ultrasound image; and e) adjusting a brightness and a contrast of the 3D ultrasound image based on the image parameters.

According to another aspect of the present invention, there is provided an ultrasound diagnostic system, comprising: an ultrasound image creating unit for creating 3D ultrasound image data based on ultrasound echo signals to form a 3D ultrasound image; an image control parameter setting unit for setting image control parameters for controlling the 3D ultrasound image; and an image processing unit for processing the 3D ultrasound image based on the image control parameters set by the image control parameter setting unit.

According to the present invention, the 3D ultrasound image can be optimized by automatically controlling the brightness and contrast of the 3D ultrasound image. Thus, a system user can conduct a diagnosis in a convenient and efficient manner.

The above and other objects and features of the present invention will become apparent from the following description of preferred embodiments given in conjunction with the accompanying drawings, in which:

Fig. 1 is a block diagram showing an ultrasound diagnostic system constructed in accordance with a preferred embodiment of the present invention;
Fig. 2 is a block diagram showing an image processor constructed in accordance with a preferred embodiment of the present invention;
Fig. 3 is a schematic diagram showing a ray casting method in accordance with a preferred embodiment of the present invention;
Fig. 4 is a flow chart showing a process of automatically optimizing a three-dimensional (3D) ultrasound image in accordance with a preferred embodiment of the present invention;
Fig. 5 is a flow chart showing a process of setting a critical value for adjusting the brightness of a 3D ultrasound image based on 3D ultrasound image data in accordance with a preferred embodiment of the present invention;
Fig. 6 is an exemplary graph showing average intensities at sampling points according to depth in accordance with a preferred embodiment of the present invention;

Fig. 7 is an exemplary graph showing an opacity transfer function in accordance with a preferred embodiment of the present invention;

Fig. 8 is a flow chart showing a process of creating the 3D ultrasound image by rendering the 3D ultrasound image data based on a critical value in accordance with a preferred embodiment of the present invention;

Fig. 9 is a flow chart showing a process of setting image parameters for optimizing the 3D ultrasound image by analyzing a histogram of the 3D ultrasound image in accordance with a preferred embodiment of the present invention;

Fig. 10 is an exemplary graph showing a relationship between the intensity of pixels and bias for adjusting the brightness of the image in accordance with a preferred embodiment of the present invention;

Fig. 11 A shows a relationship between the output intensity and input intensity of pixels when the bias of adjusting the brightness is increased; and

Fig. 11B shows a relationship between the output intensity and input intensity of pixels when the position of adjusting the contrast is increased.

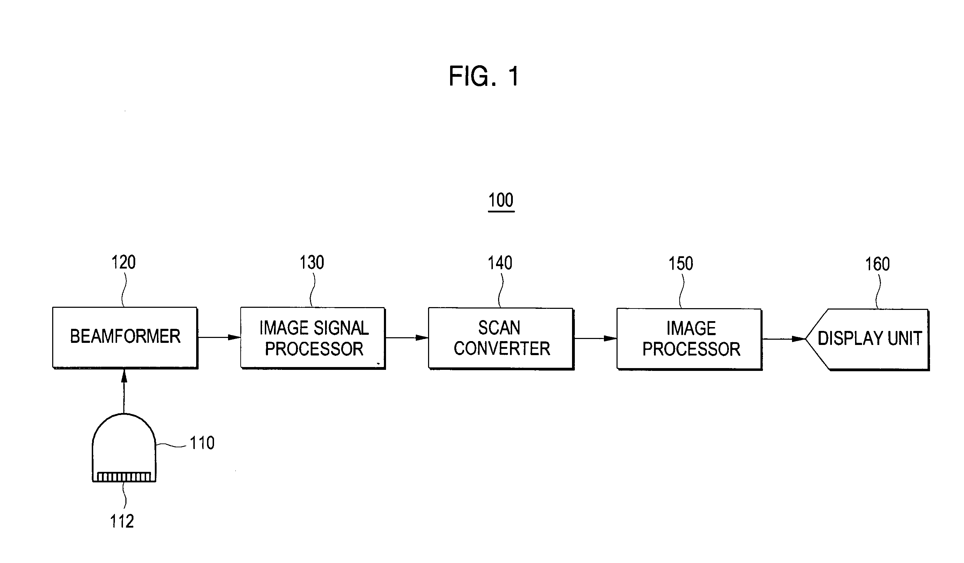

Hereinafter, a preferred embodiment of the present invention will be described with reference to Figs. 1 to 11B.

Fig. 1 is a block diagram showing an ultrasound diagnostic system constructed in accordance with a preferred embodiment of the present invention. As shown in Fig. 1, an ultrasound diagnostic system 100 includes a probe 110, a beamformer 120, an image signal processor 130, a scan converter 140, an image processor 150 and a display unit 160. The image signal processor 130 and image processor 150 may be implemented by using a single processor.

The probe 110 includes a one-dimensional or two-dimensional (2D) transducer array 112 having a plurality of transducer elements. By properly delaying the pulses applied to the transducer elements, the probe 110 transmits a focused ultrasound beam to a target object (not shown) along a transmit scan line. Ultrasound echo signals reflected from a focal point (not shown) on the transmit scan line are received by the transducer elements at different times. The transducer elements convert the received ultrasound echo signals to electrical receive signals, which are supplied to the beamformer 120. The beamformer 120 appropriately delays the electrical receive signals supplied from the transducer array 112 and then sums the delayed receive signals to provide a receive beam indicating a reflected ultrasound energy level.

For example, the image signal processor 130, a digital signal processor (DSP), performs an envelope detection on the receive signals to detect the intensities thereof. It then produces ultrasound image data based on the position information of multiple points on each scan line and data obtained from each point. The ultrasound image data include x and y coordinates of the points, an angle between a vertical scan line and each scan line and the like. Further, the image signal processor 130 produces 3D ultrasound image data of the target object by using 2D ultrasound image data. The 3D ultrasound image data represented in conical coordinates are scan-converted into 3D ultrasound image data represented in the Cartesian coordinates in the scan converter 140.

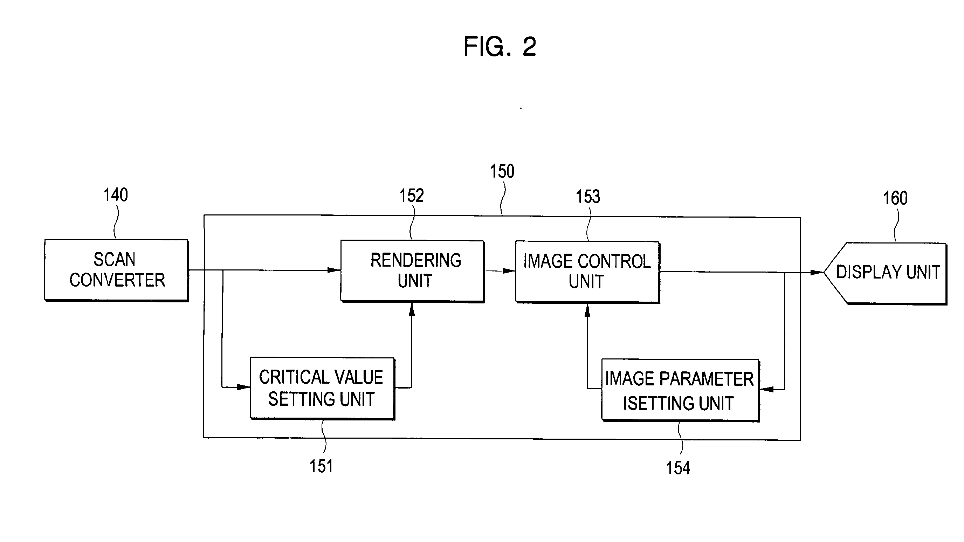

The image processor 150 creates a 3D ultrasound image based on the 3D ultrasound image data and optimizes the 3D ultrasound image by setting image control parameters for adjusting the brightness and contrast of the 3D ultrasound image. The image control parameters include a critical value for rendering the 3D ultrasound image data and image parameters for the 3D ultrasound image. As shown in Fig. 2, the image processor 150 includes a critical value setting unit 151, a rendering unit 152, an image control unit 153 and an image parameter setting unit 154.

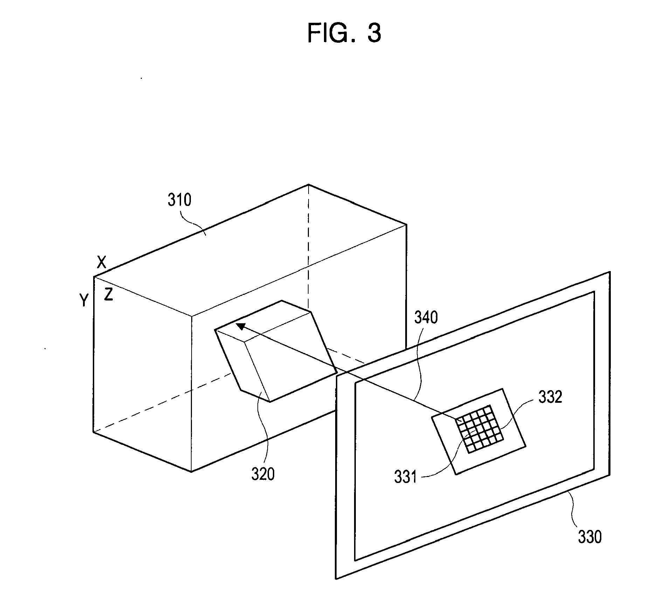

As shown in Fig. 3, the critical value setting unit 151 selects a central pixel 331 and a specified number of adjacent pixels 332 to the central pixel 331 (e.g., 5 x 5 pixels) on a viewing plane 330 including multiple pixels. It then projects an imaginary ray 340 from each of the pixels 331 and 332 to the volume data 320 in a volume space 310. Then, the critical value setting unit 151 performs sampling at specified sampling intervals along the imaginary ray 340 to a predetermined depth and detects the intensities at sampling points, respectively. After calculating an average of the intensities at sampling points of the same sampling order, a minimum average intensity is set as a critical value for distinguishing an object area from an empty area. The viewing plane 330 corresponds to a screen of the display unit 160 (on which the 3D ultrasound image is displayed) and the volume space 310 is a 3D space extended from the viewing plane 330. Further, the volume data 320 are positioned in the volume space 310 through the scan conversion in the scan converter 140 and include an object area to be imaged and an empty area not to be imaged. For instance, in case of the fetus, amniotic liquid corresponds to the empty area and the face of the fetus corresponds to the object area.

The rendering unit 152 renders the 3D ultrasound image data outputted from the scan converter 140 based on the critical value, which is set by the critical value setting unit 151. The image control unit 153 controls the brightness and contrast of the 3D ultrasound image outputted from the rendering unit 152 based on the image parameters. The image parameter setting unit 154 analyzes a histogram of the 3D ultrasound image outputted from the image control unit 153. It then sets the image parameters for adjusting the brightness and contrast of the 3D ultrasound image based on analysis results.

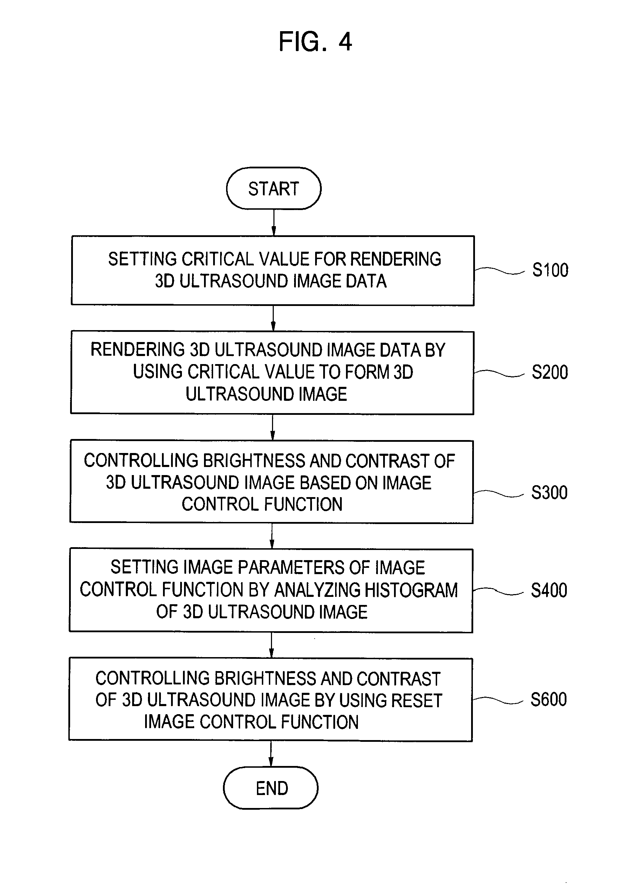

Hereinafter, the operations of the image processor 150 will be described in detail with reference to Figs. 4 to 11B.

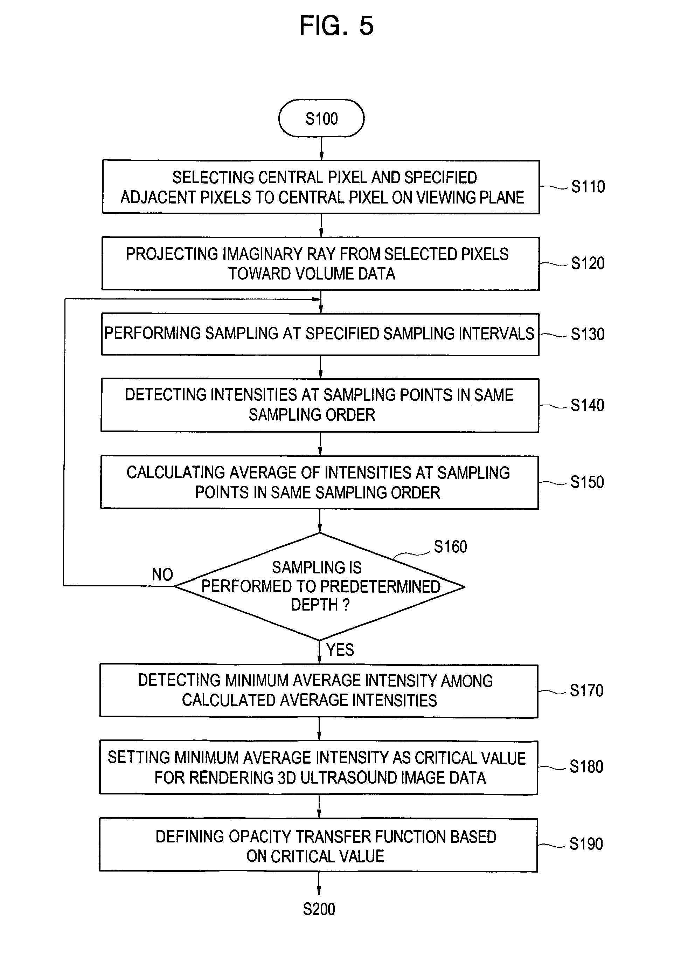

Fig. 4 is a flow chart showing a process of automatically optimizing the 3D ultrasound image in accordance with a preferred embodiment of the present invention. Referring now to Fig. 4, at step S 100, the critical value setting unit 151 of the image processor 150 sets a critical value for rendering the 3D ultrasound image data outputted from the scan converter 140. A detailed description of step S100 is provided with reference to Fig. 5.

Fig. 5 is a flow chart showing a process of setting a critical value for rendering the 3D ultrasound image data in accordance with a preferred embodiment of the present invention. Referring now to Fig. 5, the critical value setting unit 151 selects the central pixel 331 and a specified number of the adjacent pixels 332 to the central pixel 331 on the viewing plane 330 containing multiple pixels at step S110. The critical value setting unit 151 projects the imaginary ray 340 from each of the selected pixels 331 and 332 toward the volume data 320 at step S120.

Next, the critical value setting unit 151 performs sampling at specified sampling intervals along the imaginary ray 340 at step S 130. It then detects the intensities at the sampling points of the same sampling order at step S140. The sampling order represents the number of sampling points counted until reaching the present sampling point from the viewing plane 330. The critical value setting unit 151 calculates an average of the intensities at sampling points of the same sampling order at step S 150. That is, the average intensity is obtained by dividing a sum of the intensities at the sampling points of the same sampling order by the number of the sampling points. Then, the critical value setting unit 151 checks whether sampling has been performed to a predetermined depth at step S160. If it is determined that sampling has not been performed to the predetermined depth, then the process returns to step S130.

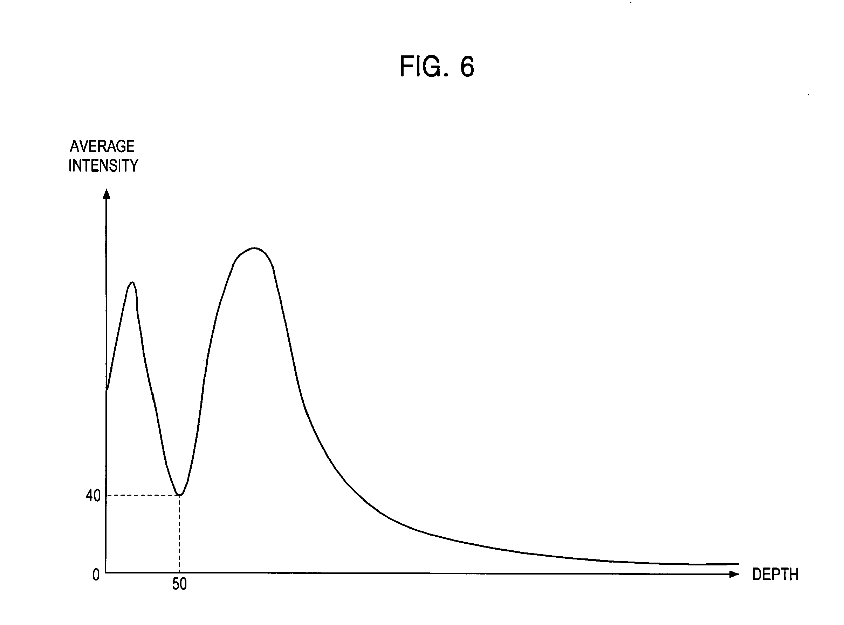

On the other hand, if it is determined that sampling has been performed to the predetermined depth, then the critical value setting unit 151 detects a minimum average intensity among the calculated average intensities at step S170. The minimum average intensity is then set as a critical value for rendering the 3D ultrasound image data at step S180. For example, as shown in Fig. 6, when the average intensity has a minimum value of 40 after sampling has been performed to the predetermined depth, the minimum value of 40 in the average intensity is set as the critical value. In this case, amniotic liquid exists at a depth of about 50 where the minimum intensity is 40, a layer of fat at a depth smaller than about 50 and the fetus at a depth greater than about 50.

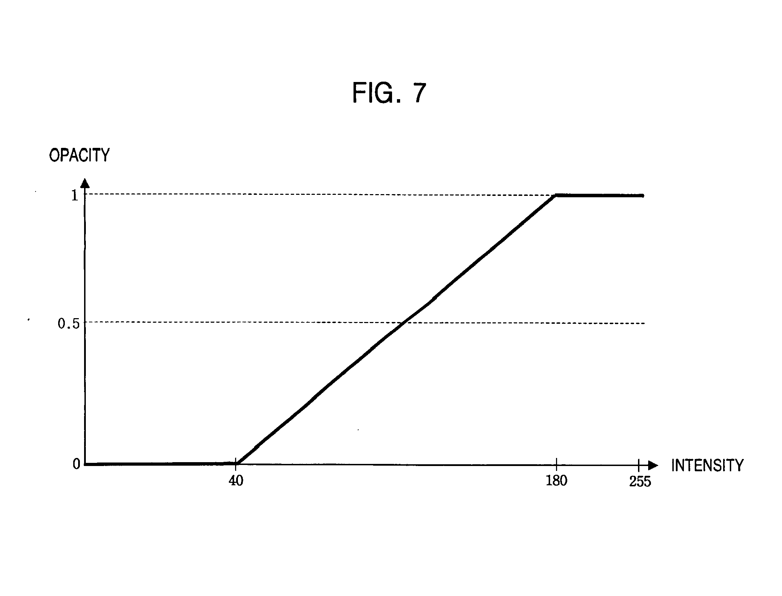

Thereafter, the critical value setting unit 151 defines an opacity transfer function based on the critical value at step S 190. The opacity transfer function represents a relationship between opacity and intensity. For example, in the above case wherein the critical value is 40 (as shown in Fig. 7): the opacity value becomes 0 at intensities ranging from 0 to 40; the opacity value varies linearly at intensities ranging from 40 to 180; and the opacity value becomes 1 at intensities ranging from 180 to 255. Accordingly, the fetus can become distinguishable by applying the opacity value only to the fetus.

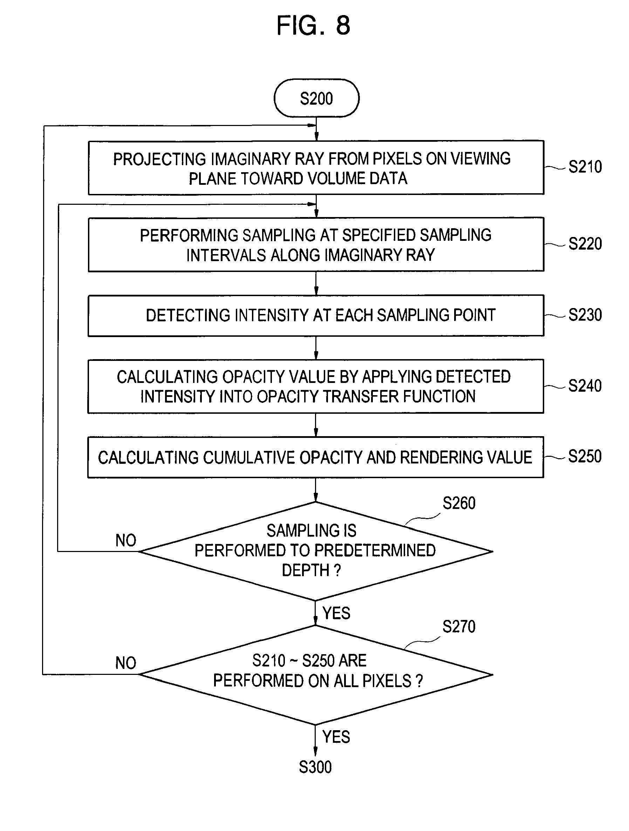

Referring now back to Fig. 4, the rendering unit 152 renders the 3D ultrasound image data based on the critical value, which is set by the critical value setting unit 151 to thereby create a 3D ultrasound image at step S200. A detailed description of step S200 is provided with reference to Fig. 8.

Fig. 8 is a flow chart showing a process of creating the 3D ultrasound image by rendering the 3D ultrasound image data based on the critical value in accordance with a preferred embodiment of the present invention. Referring to Fig. 8, the rendering unit 152 projects the imaginary ray 340 from each of the specified pixels on the viewing plane 330 including multiple pixels toward the volume data 320 at step S210. Further, the rendering unit 152 performs sampling at specified sampling intervals along the imaginary ray 340 at step S220. Then, the rendering unit 152 detects the intensity at each sampling point at step S230 and an opacity value is calculated by applying the detected intensity into the opacity transfer function at step S240.

Thereafter, the rendering unit 152 calculates a cumulative opacity and rendering value based on the intensities and opacities at the sampling points at step S250. A cumulative opacity R is obtained by combining the opacities at the sampling points, as shown by the following equation (1):

$$ R = (1-A_1)(1-A_2)\cdots(1-A_{n-1}) \qquad (1) $$

wherein $A_{n-1}$ is the opacity at $(n-1)^{th}$ sampling point. Further, the rendering value D is obtained by combining the intensities, opacities and cumulative opacities at the sampling points, as shown by the following equation (2):

$$ D = C_1A_1 + C_2A_2(1-A_1) + C_3A_3(1-A_1)(1-A_2) + \cdots $$
$$ + C_nA_n(1-A_1)(1-A_2)\ldots(1-A_{n-1}) \qquad (2) $$

wherein $C_n$ is the intensity at $n^{th}$ sampling point.

The rendering unit 152 checks whether the sampling has been performed to a predetermined depth at step S260. If it is determined that the sampling has not been performed to the predetermined depth, then the process returns to step S220. On the other hand, if it is determined that sampling has been performed to the predetermined depth, then the rendering unit 152 checks whether the procedure at steps S210 to S250 has been performed on all the pixels on the viewing plane 330 at step S270. If it is determined that the procedure has not been performed on all the pixels, then the process returns to step S210. On the other hand, if it is determined that the procedure has not been performed on all the pixels, then the process proceeds to step S300 set forth in Fig. 4.

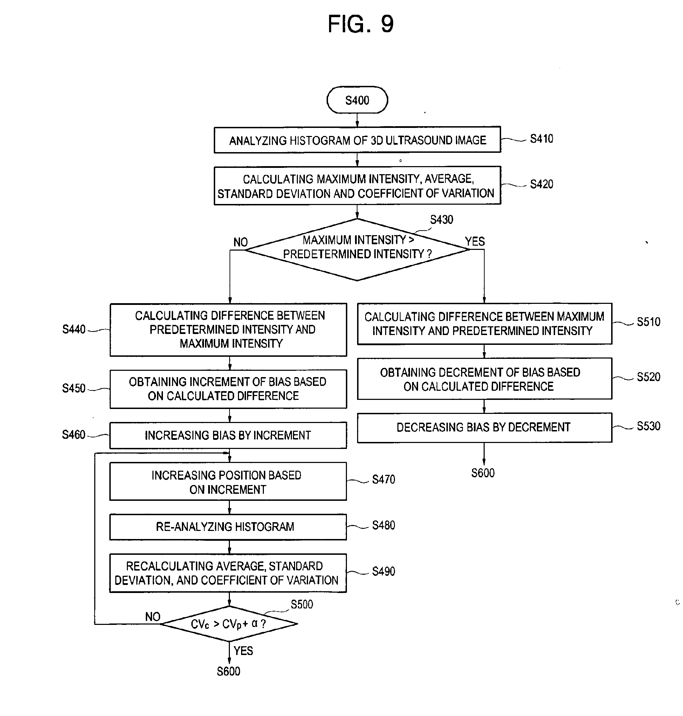

Referring back to Fig. 4, the image control unit 153 controls the brightness and contrast of the 3D ultrasound image based on an image control function at step S300. The image control function contains the image parameters including a bias parameter for adjusting the brightness of the ultrasound image and a position parameter for adjusting the contrast of the ultrasound image, wherein the image parameters have basic set values that are zero. Next, the image parameter setting unit 154 sets the image parameters for optimizing the 3D ultrasound image by analyzing a histogram of the 3D ultrasound image outputted from the image control unit 153 at step S400. A detailed description of step S400 is provided with reference to Fig. 9.

Fig. 9 is a flow chart showing a process of setting the image parameters for optimizing the 3D ultrasound image by analyzing a histogram of the 3D ultrasound image in accordance with a preferred embodiment of the present invention. Referring to Fig. 9, the image parameter setting unit 154 analyzes a histogram of the 3D ultrasound image outputted from the image control unit 153 at step S410. It then calculates a maximum intensity, an average (mean), a standard deviation and a coefficient of variation at step S420. The coefficient of variation (CV), which is defined as a ratio of the standard deviation to the mean, measures the spread of a set of data as a proportion of its mean. Then, the image parameter setting unit 154 checks whether the calculated maximum intensity is greater than a predetermined intensity at step S430.

If it is determined that the calculated maximum intensity is smaller than the predetermined intensity, then the image parameter setting unit 154 calculates a difference between the predetermined intensity and the maximum intensity at step S440. An increment of bias is obtained based on the calculated difference at step S450 and the bias is increased by the increment at step S460. The image parameter setting unit 154 also increases the position based on the increment at step S470

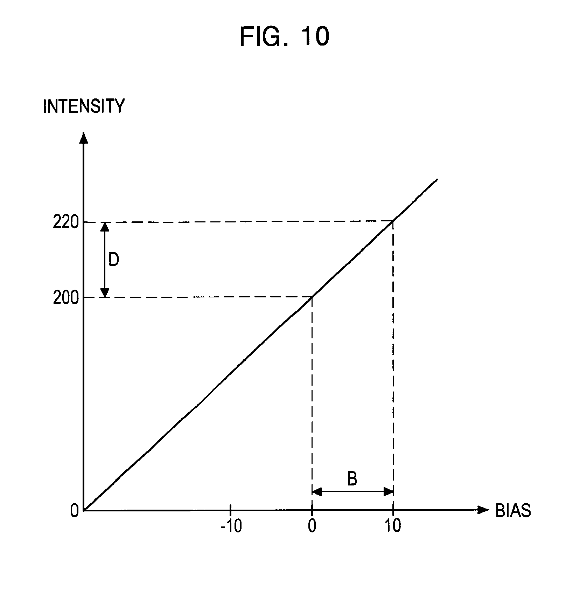

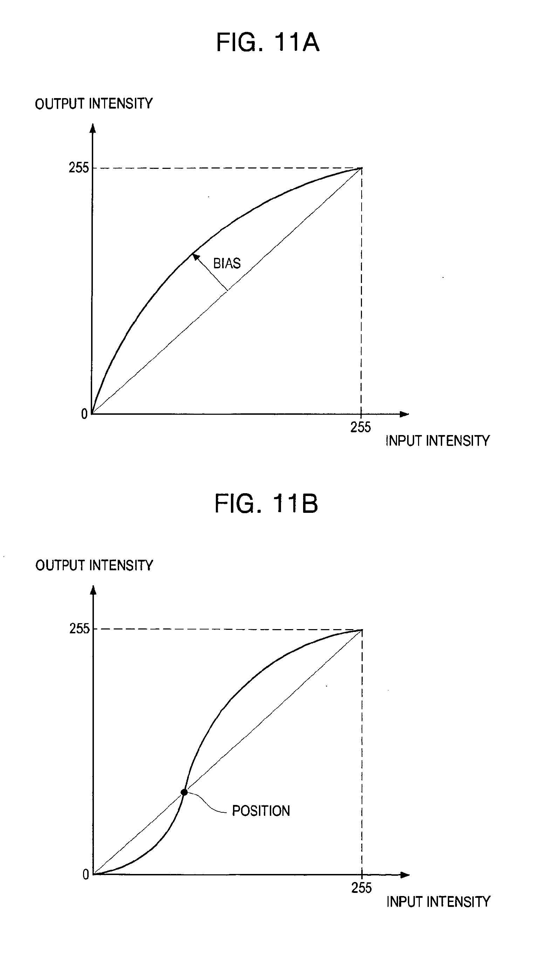

Fig. 10 shows a calculation of the increment of bias, wherein a difference (D=20) between the predetermined intensity (220) and the maximum intensity (200) is calculated to determine the increment of bias (B=10) corresponding to the difference (D=20). Fig. 11A shows the output intensity versus the input intensity of pixels when the bias is increased, wherein the output intensities are varied along an exponential curve to thereby increase the average brightness of the image. Fig. 11B depicts the output intensity versus the input intensity of pixels when the position is increased, wherein the output intensities are varied along logarithmical and exponential curves before and after the position, respectively, thereby enhancing the contrast of the image. That is, since a standard deviation is almost constant and the average brightness is decreased as the position is increased, the coefficient of variation is increased to enhance the contrast of the image.

Thereafter, the histogram is reanalyzed at step S480. Further, the average, standard deviation and coefficient of variation are recalculated at step S490. The image parameter setting unit 154 checks whether the recalculated coefficient of variation $CV_C$ is greater than the sum of a fixed value $\alpha$ and the previous coefficient of variation $CV_P$ obtained in step S420 by comparing them with each other at step S500. If it is determined that the recalculated coefficient of variation $CV_C$ is smaller than the sum of a fixed value $\alpha$ and the previous coefficient of variation $CV_P$, then the process returns to step S470. If it is not, however, then the process proceeds to step S600.

Further, if it is determined that the maximum intensity is greater than the predetermined intensity in step S430, then the image parameter setting unit 154 calculates a difference between the maximum intensity and the predetermined intensity at step S510. Further, a decrement of bias is obtained based on the calculated difference at step S520 and the bias is decreased by the decrement at step S530. In this case (opposite to Fig. 11A), the output intensities are varied along a logarithmical curve to thereby decrease the average brightness of the image.

Referring back to Fig. 4, the image control unit 153 applies the image parameters (bias and position) outputted from the image parameter setting unit 154 to the image control function. It then controls the brightness and contrast of the 3D ultrasound image by using the image control function at step S600.

While the present invention has been described and illustrated with respect to a preferred embodiment of the invention, it will be apparent to those skilled in the art that variations and modifications are possible without deviating from the broad principles and teachings of the present invention, which should be limited solely by the scope of the claims appended hereto.

**Claims**

1. A method of automatically controlling a brightness and a contrast of a three-dimensional (3D) ultrasound image, comprising the steps of:

a) creating 3D ultrasound image data based on ultrasound echo signals;
b) setting a critical value for rendering the 3D ultrasound image data;
c) rendering the 3D ultrasound image data by using the critical value to form the 3D ultrasound image;
d) analyzing a histogram of the 3D ultrasound image to set image parameters for the 3D ultrasound image; and
e) adjusting the brightness and the contrast of the 3D ultrasound image based on the image parameters.

2. The method of Claim 1, wherein the step b) includes:

b1) producing volume data based on the ultrasound echo signals;
b2) projecting an imaginary ray toward the volume data and performing sampling at specified sampling intervals along the imaginary ray;
b3) calculating average intensities, each average intensity being at sampling points of a same sampling order; and
b4) setting the critical value based on the calculated average intensities.

3. The method of Claim 2, wherein the step b2) includes:

b21) selecting a central pixel and a specified number of adjacent pixels to the central pixel among multiple pixels formed on a viewing plane disposed away from an imaginary space containing the volume data;
b22) projecting an imaginary ray from each of the selected pixels toward the volume data; and
b23) performing sampling at specified sampling intervals along the imaginary ray and calculating intensities at sampling points.

4. The method of Claim 2, wherein the step b4) includes:

b41) detecting a minimum average intensity among the average intensities; and
b42) setting the minimum average intensity as a critical value.

5. The method of Claim 3, wherein the step c) includes:

c1) projecting an imaginary ray from each of pixels formed on the viewing plane toward the volume data;
c2) performing sampling at specified sampling intervals along the imaginary ray and calculating intensities at sampling points;
c3) calculating opacities corresponding to the intensities at the sampling points based on the critical value; and
c4) calculating rendering values based on the intensities and the opacities.

6. The method of Claim 1, wherein the image parameters include a first image parameter for adjusting the brightness of the 3D ultrasound image and a second image parameter for adjusting the contrast of the 3D ultrasound image.

7. The method of Claim 6, wherein the step d) includes:

d1) analyzing a histogram of the 3D ultrasound image and calculating an average, a standard deviation, a maximum intensity and a coefficient of variation based on analysis results;
d2) setting the first image parameter by comparing the maximum intensity with a predetermined intensity; and
d3) setting the second image parameter by reanalyzing the histogram of the 3D ultrasound image.

8. The method of Claim 7, wherein the step d2) includes:

d21) if it is determined that the maximum intensity is smaller than the predetermined intensity, calculating a difference between the maximum intensity and the predetermined intensity;
d22) obtaining an increment of the first image parameter based on the calculated difference; and
d23) increasing the first image parameter by the increment.

9. The method of Claim 7, wherein the step d2) includes:

d24) if it is determined that the maximum intensity is greater than the predetermined intensity, calculating a difference between the maximum intensity and the predetermined intensity;
d25) obtaining a decrement of the first image parameter based on the calculated difference; and
d26) decreasing the first image parameter by the decrement.

**10.** The method of Claim 8, wherein the step d3) includes:

d31) increasing the second image parameter based on the increment of the first image parameter;
d32) reanalyzing a histogram of the 3D ultrasound image and recalculating an average, a standard deviation, a maximum intensity and a coefficient of variation; and
d33) setting the second image parameter based on the coefficient of variation calculated in the step d1) and the coefficient of variation recalculated in the step d32).

**11.** An ultrasound diagnostic system, comprising:

an ultrasound image creating unit for creating 3D ultrasound image data based on ultrasound echo signals to form a 3D ultrasound image;
an image control parameter setting unit for setting image control parameters for controlling the 3D ultrasound image; and
an image processing unit for processing the 3D ultrasound image based on the image control parameters set by the image control parameter setting unit.

**12.** The ultrasound diagnostic system of Claim 11, wherein the image control parameter setting unit includes:

a critical value setting unit for setting a critical value for rendering the 3D ultrasound image data; and
an image parameter setting unit for setting image parameters for adjusting a brightness and a contrast of the 3D ultrasound image.

**13.** The ultrasound diagnostic system of Claim 12, wherein the critical value setting unit includes:

a unit for selecting a central pixel and a specified number of adjacent pixels to the central pixel among multiple pixels formed on a viewing plane disposed away from an imaginary space containing volume data produced based on the ultrasound echo signals, the unit being configured to project an imaginary ray from each of the selected pixels toward the volume data;
a unit for performing sampling at specified sampling intervals along the imaginary ray and detecting intensities at sampling points; and
a unit for setting the critical value based on the detected intensities.

**14.** The ultrasound diagnostic system of Claim 12, wherein the image parameter setting unit includes:

a first image parameter setting unit for setting a first image parameter for adjusting the brightness of the 3D ultrasound image; and
a second image parameter setting unit for setting a second image parameter for adjusting the contrast of the 3D ultrasound image.

**15.** The ultrasound diagnostic system of Claim 14, wherein the first image parameter setting unit includes:

a unit for analyzing a histogram of the 3D ultrasound image to calculate an average, a standard deviation, a maximum intensity and a coefficient of variation based on analysis results; and
a unit for setting the first image parameter by comparing the maximum intensity with a predetermined intensity.

**16.** The ultrasound diagnostic system of Claim 14, wherein the second image parameter setting means includes:

a unit for reanalyzing a histogram of the 3D ultrasound image to recalculate an average, a standard deviation, a maximum intensity and a coefficient of variation based on reanalysis results; and
a unit for setting the second image parameter based on the recalculated coefficient of variation.

# FIG. 1

100

| 120 | 130 | 140 | 150 | 160 |
|---|---|---|---|---|
| BEAMFORMER | IMAGE SIGNAL PROCESSOR | SCAN CONVERTER | IMAGE PROCESSOR | DISPLAY UNIT |

110

112

EP 1 750 143 A2

# FIG. 2

EP 1 750 143 A2

# FIG. 3

# FIG. 4

START

SETTING CRITICAL VALUE FOR RENDERING
3D ULTRASOUND IMAGE DATA — S100

RENDERING 3D ULTRASOUND IMAGE DATA BY
USING CRITICAL VALUE TO FORM 3D
ULTRASOUND IMAGE — S200

CONTROLLING BRIGHTNESS AND CONTRAST OF
3D ULTRASOUND IMAGE BASED ON IMAGE
CONTROL FUNCTION — S300

SETTING IMAGE PARAMETERS OF IMAGE
CONTROL FUNCTION BY ANALYZING HISTOGRAM
OF 3D ULTRASOUND IMAGE — S400

CONTROLLING BRIGHTNESS AND CONTRAST
OF 3D ULTRASOUND IMAGE BY USING RESET
IMAGE CONTROL FUNCTION — S600

END

# FIG. 5

S100

SELECTING CENTRAL PIXEL AND SPECIFIED ADJACENT PIXELS TO CENTRAL PIXEL ON VIEWING PLANE — S110

PROJECTING IMAGINARY RAY FROM SELECTED PIXELS TOWARD VOLUME DATA — S120

PERFORMING SAMPLING AT SPECIFIED SAMPLING INTERVALS — S130

DETECTING INTENSITIES AT SAMPLING POINTS IN SAME SAMPLING ORDER — S140

CALCULATING AVERAGE OF INTENSITIES AT SAMPLING POINTS IN SAME SAMPLING ORDER — S150

S160 — SAMPLING IS PERFORMED TO PREDETERMINED DEPTH ?

NO

YES

DETECTING MINIMUM AVERAGE INTENSITY AMONG CALCULATED AVERAGE INTENSITIES — S170

SETTING MINIMUM AVERAGE INTENSITY AS CRITICAL VALUE FOR RENDERING 3D ULTRASOUND IMAGE DATA — S180

DEFINING OPACITY TRANSFER FUNCTION BASED ON CRITICAL VALUE — S190

S200

# FIG. 6

# FIG. 7

# FIG. 8

```
           ( S200 )
              │
              ▼
┌──────────────────────────────────────────────┐
│ PROJECTING IMAGINARY RAY FROM PIXELS ON VIEWING│ ── S210
│ PLANE TOWARD VOLUME DATA                        │
└──────────────────────────────────────────────┘
              │
              ▼
┌──────────────────────────────────────────────┐
│ PERFORMING SAMPLING AT SPECIFIED SAMPLING      │ ── S220
│ INTERVALS ALONG IMAGINARY RAY                  │
└──────────────────────────────────────────────┘
              │
              ▼
┌──────────────────────────────────────────────┐
│ DETECTING INTENSITY AT EACH SAMPLING POINT     │ ── S230
└──────────────────────────────────────────────┘
              │
              ▼
┌──────────────────────────────────────────────┐
│ CALCULATING OPACITY VALUE BY APPLYING DETECTED │ ── S240
│ INTENSITY INTO OPACITY TRANSFER FUNCTION       │
└──────────────────────────────────────────────┘
              │
              ▼
┌──────────────────────────────────────────────┐
│ CALCULATING CUMULATIVE OPACITY AND RENDERING VALUE │ ── S250
└──────────────────────────────────────────────┘
              │
              ▼
        S260
   ◇ SAMPLING IS
NO ◇ PERFORMED TO PREDETERMINED
   ◇ DEPTH ?
              │ YES
              ▼
        S270
   ◇ S210 ~ S250 ARE
NO ◇ PERFORMED ON ALL PIXELS ?
              │ YES
              ▼
            S300
```

# FIG. 9

S400

ANALYZING HISTOGRAM OF 3D ULTRASOUND IMAGE — S410

CALCULATING MAXIMUM INTENSITY, AVERAGE, STANDARD DEVIATION AND COEFFICIENT OF VARIATION — S420

S430
MAXIMUM INTENSITY > PREDETERMINED INTENSITY ?
NO      YES

S440 — CALCULATING DIFFERENCE BETWEEN PREDETERMINED INTENSITY AND MAXIMUM INTENSITY

CALCULATING DIFFERENCE BETWEEN MAXIMUM INTENSITY AND PREDETERMINED INTENSITY — S510

S450 — OBTAINING INCREMENT OF BIAS BASED ON CALCULATED DIFFERENCE

OBTAINING DECREMENT OF BIAS BASED ON CALCULATED DIFFERENCE — S520

S460 — INCREASING BIAS BY INCREMENT

DECREASING BIAS BY DECREMENT — S530

INCREASING POSITION BASED ON INCREMENT — S470

S600

RE-ANALYZING HISTOGRAM — S480

RECALCULATING AVERAGE, STANDARD DEVIATION, AND COEFFICIENT OF VARIATION — S490

S500
$CV_c > CV_p + \alpha$ ?
NO

YES
S600

16

# FIG. 10

# FIG. 11A

OUTPUT INTENSITY

255

BIAS

0

255

INPUT INTENSITY

# FIG. 11B

OUTPUT INTENSITY

255

POSITION

0

255

INPUT INTENSITY